# EUROPÄISCHE PATENTANMELDUNG

(11) **EP 0 885 624 A2**
(43) Veröffentlichungstag der Anmeldung: **23.12.1998**
(21) Anmeldenummer: 98111344.2
(22) Anmeldetag: 19.06.1998
(51) Int. Cl.: A61M 25/088, A61M 25/092

(54) **Steuerbare Führungskatheteranordnung**

(30) Priorität: 19.06.1997 DE 19726093
(71) Anmelder: Lascor GmbH, 81456 München (DE)
(72) Erfinder: Weber, Dietmar, 82061 Neuried (DE)
(74) Vertreter: Ruschke, Hans Edvard, Dipl.-Ing.

(57) **Zusammenfassung**

Flexibler Führungskatheter mit einem vorgeformten und mit einem Arbeitslumen zum Vorschieben von Werkzeugen versehenen Innenkatheter und einem Außenschlauch, der dicker und starrer als der Innenkatheter ist und aufgrund seines Umfangs an der Eintrittsöffnung des Organs abgestützt werden kann, wobei der vorgeformte Innenkatheter sich innerhalb des Außenschlauches dessen Form anpaßt und außerhalb des Außenschlauchs im Organ wieder seine vorgeformte Gestalt annimmt, wodurch sich je nach Vorschub des Innenkatheters verschiedene Krümmungen realisieren lassen und wobei der Außenschlauch bei Vorschub eine Kippbewegung um die Eintrittsöffnung des Organs ausführt, sodaß dort eine stabile Drehachse entsteht, sodaß stufenlos jeder Punkt eines dreidimensionalen Raumes ohne Austausch des Arbeitsinstrumentes erreicht werden kann.

## Beschreibung

Die vorliegende Erfindung betrifft eine flexible Führungskatheteranordnung mit verbesserter Steuerbarkeit des distalen Endabschnittes.

Führungskatheter werden in vielen Bereichen der Medizin eingesetzt. So ist z.B. in der DE 37 18 139 C1 ein Herzkatheter in Form eines Schlauches offenbart, dessen distales Ende als Sonde zum Lokalisieren pathologischer Areale mit einer Elektrodenanordnung versehen ist. Um bestimmte Areale z.B. im Herzen gezielt und schnell in orthogonaler Orientierung ansteuern zu können, ist ein spezieller Führungskatheter erforderlich. Zu diesem Zweck ist in der Literaturstelle "*Suppl. 1 zur Zeitschrift für Kardiologie, Band 83 (1994)*" ein Führungskatheter mit zwei aufeinander senkrecht stehenden Krümmungsebenen offenbart. So konnten bei Verwendung unterschiedlicher Kombinationen von Krümmungsradien und -winkeln gezielt alle Endokardareale erreicht werden.

Der Nachteil bei diesem Katheter besteht darin, daß er aufgrund seiner vorbestimmten Form nur eingeschränkt einsetzbar ist. Sobald eine andere Kombination aus Krümmungsradien und -winkeln erwünscht ist, muß ein neuer Katheter eingesetzt werden. Ein weiterer Nachteil ist das durch die vorgegebene Krümmung erschwerte Einführen eines Arbeitsinstrumentes.

In der DE 93 14 543 U1 wird ein flexibler Führungskatheter offenbart mit einem Arbeitskanal und einem schlauchförmigen Mantel, der eine schraubenförmige Außenwicklung mit definiertem Steigungswinkel aus nicht dehnbarem Material sowie eine sich in Längsrichtung erstreckende Kammer aufweist. Wird in diese Kammer eine flüssiges Medium eingebracht, verschieben sich die Windungen aufgrund dieser radialen Druckbeaufschlagung in verschiedenen Bereichen in unterschiedlicher Weise relativ zueinander. Durch diese in Umfangsrichtung ungleichmäßige Verschiebung wird eine Abwinklung des Führungskatheters hervorgerufen. Wird die Druckbelastung anschließend wieder reduziert, wirkt die Elastizität des Mantels als Rückstellkraft und der Führungskatheter nimmt wieder seine ursprüngliche Gestalt an. Daraus ergibt sich zunächst der Nachteil, das diese Konstruktion durch ihre Verwendung einer mit einem flüssigen Medium zu füllenden Kammer sehr aufwendig ist Weiterhin ist von Nachteil, daß der Katheter aufgrund dieser Konstruktion einen relativ großen Außendurchmesser aufweist, was dazu führt, daß das distale Ende z.B. kaum über das coronare Ostium hinaus vorgeschoben werden kann. Schließlich besteht nach wie vor der Nachteil, daß der Katheter aufgrund des definierten Außenwinkels der Steigung der Wicklung nur sehr eingeschränkt einsetzbar ist, d.h. man ist weit davon entfernt, alle gewünschten Verbiegungen zu erzielen, um alle Punkte eines dreidimensionalen Raumes im Bereich des distalen Endes des Führungskatheters erreichen zu können.

Die DE 91 00 016 U1 lehrt einen Führungskatheter mit ausreichend kleinem Außendurchmesser, indem durch den Längsdurchgang des Führungskatheters nicht gleich die Werkzeuge vorgeschoben werden, sondern zunächst ein zweiter Führungskatheter. Dieser zweite Führungskatheter - in dem dann die Werkzeuge vorgeschoben werden - weist für seine Biegbarkeit keine geflochtene Schicht mehr auf wie der erste, da er den ersten spurt. Somit ist er so dünnwandig, daß sein Außendurchmesser niedriger ist und er auch über das coronare Ostium hinaus vorgeschoben werden kann. Damit ist ein Nachteil der zuvor diskutierten Druckschrift vermieden. Allen genannten Druckschriften ist aber nach wie vor der Nachteil gemeinsam, daß nur eingeschränkte Verformungen des Katheters aufgrund der geflochtenen Schicht möglich sind.

Demgegenüber ist es ein Ziel der vorliegenden Erfindung, einen Katheter bereitzustellen, der fast perfekt steuerbar ist, d.h. der am distalen Ende verschiedene Krümmungsradien und -winkel bei ein und demselben Katheter zuläßt, ohne daß ein Katheterwechsel erforderlich ist. Darüber hinaus ist es ein Ziel, eine Einrichtung zu schaffen, die viele verschiedene Dreh- bzw. Kippbewegungen des Katheters um eine weitgehend feste Achse ermöglicht, um wirklich alle Punkte eines dreidimensionalen Raumes im Bereich des distalen Endes des Katheters zu erreichen, also z. B. in einem Hohlorgan.

Die Aufgabe der Erfindung wird mit den Merkmalen des Anspruchs 1 gelöst. Es wird ein flexibler Führungskatheter mit einem vorgeformten und mit einem Arbeitslumen zum Vorschieben von Werkzeugen versehenen Innenkatheter vorgesehen, wobei der Innenkatheter mit mindestens einem Außenschlauch versehen ist, der dicker und steifer als der Innenkatheter ist und aufgrund seines Umfangs an der Eintrittsöffnung in ein Organ abgestützt werden kann, wobei der vorgeformte Innenkatheter sich innerhalb des Außenschlauches dessen Form anpaßt und gestreckt ist, jedoch außerhalb des Außenschlauchs im Organ wieder seine vorgeformte Gestalt annimmt. Dadurch lassen sich je nach Vorschubposition des Innenkatheters verschiedene Krümmungen realisieren, wobei der Außenschlauch bei weiterem Vorschieben eine Kippbewegung um die Eintrittsöffnung des Organs ausführt, so daß dort eine stabile Drehachse entsteht und stufenlos jeder Punkt eines dreidimensionalen Raumes ohne Austausch des Arbeitsinstrumentes erreicht werden kann.

Ein Vorteil der Erfindung besteht darin, daß sich durch die Verwendung eines flexiblen aber mit verschiedenen Krümmungsradien und -ebenen vorgeformten Innenkatheters und mindestens eines zusätzlichen dickeren, steifen und auf dem Innenkatheter anliegenden Außenschlauchs, durch den der Innenkatheter vorgeschoben wird, je nach Länge des aus dem Außenschlauch in das Organ hineinragenden Innenkatheters verschiedene Krümmungen am Innenkatheter realisieren lassen. Der Innenkatheter weist ein Arbeitslumen auf, durch das dann beliebige Arbeitswerkzeuge vorgeschoben werden können.

Ein weiterer Vorteil der Erfindung besteht darin, daß durch die Verwendung des zusätzlichen dickeren Außenschlauchs eine Einrichtung geschaffen wird, die es ermöglicht, daß der Außenschlauch an der Eintrittsöffnung zum Organ abgestützt werden kann und daß so eine Dreh- bzw. Kippbewegung des Katheters erzeugt werden kann, nämlich wenn sich der Außenschlauch beim Nachschieben desselben nicht weiter in das Organ hineinbewegt, sondern an der Eintrittsöffnung aufgehalten wird und eine Kippbewegung vollführt.

Weitere Ziele und Vorteile der vorliegenden Erfindung ergeben sich aus dem folgenden Teil dieser Beschreibung sowie den beigefügten Zeichnungen, die eine derzeit bevorzugte Ausführungsform zeigen und an denen die Prinzipien der Erfindung erläutert werden.
- Fig. 1: zeigt einen vorgeformten flexiblen Innenkatheter.
- Fig. 2a/b: zeigen die erfindungsgemäße Katheteranordnung mit dem teilweise aus dem Außenschlauch hervorragenden Innenkatheter mit verschiedener Krümmung je nach relativer Vorschubposition.
- Fig. 3a/b: zeigen die Kippbewegung des Außenschlauchs beim Nachschieben desselben gegen die Eintrittsöffnung in ein Organ.

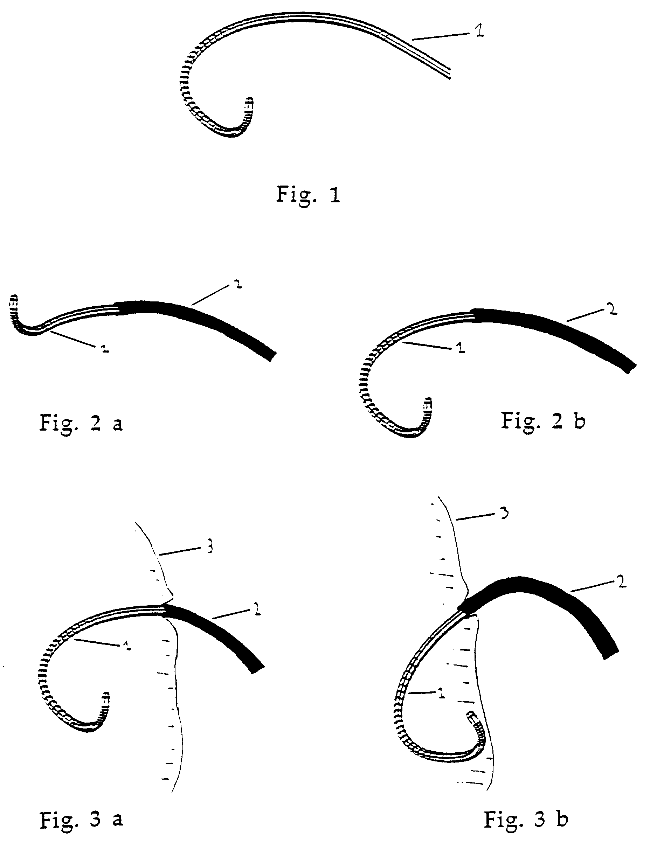

Fig. 1 stellt den vorgeformten Innenkatheter 1 dar. Er besteht aus medizinisch verträglichem elastischem Kunststoff-Material, das in einer bestimmten Weise dauerhaft vorgeformt ist, das aber aufgrund seiner Elastizität auch gestreckt oder in andere Formen gebogen werden kann, wenn äußere Kräfte angreifen. Der Innenkatheter nach Fig. 1 zeichnet sich insbesondere dadurch aus, daß der Endabschnitt des Katheters axial einwärts vom distalen Ende mit in Längsrichtung variablen Krümmungsradien und/oder in verschiedenen, z. B. zueinander senkrechten oder schrägen Krümmungsebenen derart vorgeformt und etwas steifer ausgebildet ist, daß er ohne äußere Krafteinwirkung von selbst etwa die in Fig. 1 angedeutete dreidimensionale Form oder eine ähnliche dreidimensionale Gestalt im Raum einnimmt. Wie ebenfalls aus Fig. 1 ersichtlich ist, kann andererseits der sich näher zum proximalen Ende des Innenkatheters an den gezeigten Endabschnitt anschließende (Haupt-)Teil des Katheters durchaus "gerade" aber stärker flexibel ausgeformt sein, wie dies bei herkömmlichen Kathetern in der Regel der Fall ist. Der derart "vorgeformte" und ausgebildete Innenkatheter 1 mit einem darin sich in Längsrichtung erstreckenden "Arbeitslumen" (in den Zeichnungen nicht gezeigt) wird in den im Durchmesser etwas dickeren Außenschlauch 2 oder Außenkatheter eingeführt, der biegesteifer ist als der voranstehend erläuterte vordere Endabschnitt des Innenkatheters 1, was zur Folge hat, daß der gesamte Endabschnitt sich den durch äußere Einflüsse erzwungenen Biegungen des Außenschlauches 2 anpaßt, solange der Innenkatheter sich noch im Inneren des Außenschlauches befindet. Ohne diese äußere Führung nimmt er aber seine dauerhaft vorgeformte Gestalt an (Fig. 1).

Das wird in den Figuren 2 a/b deutlich, die gleichzeitig das grundlegende Prinzip der Erfindung illustrieren. Der mit verschiedenen Krümmungsradien vorgeformte Innenkatheter 1 wird durch den dickeren und steiferen Außenschlauch 2 geführt und beim Einführen der Katheteranordnung zunächst zusammen mit diesem nach vorne vorgeschoben. Innerhalb des Außenschlauchs paßt sich die Krümmung des Innenkatheters der durch äußere Umstände gegebenen Form des Außenschlauchs an, und zwar insbesondere auch der speziell vorgeformte distale Endabschnitt des Innenkatheters. Jedoch je nach der Länge, um die der vordere Endabschnitt aus dem Außenschlauch hervorragt, nimmt der Innenkatheter axial außerhalb davon wieder mehr oder weniger seine vorgeformte Gestalt an. So lassen sich je nach der Vorschubposition des Innenkatheters im Verhältnis zum distalen Ende des Außenschlauches aufgrund der vorgeformten Gestalt des Innenkatheters verschiedene Krümmungen bzw. Positionen des distalen Endes des Innenkatheters realisieren, d. h. es können nahezu beliebige Punkte im Inneren z. B. eines Hohlorgans erreicht werden. Damit ist man im Besitz einer flexiblen bzw. sehr gut steuerbaren Katheteranordnung, ohne diese je nach der gewünschter Form des Endabschnittes wechseln zu müssen. Weiterhin ergibt sich der Vorteil, daß sich ein Katheterwechsel, falls dennoch notwendig, auf den Wechsel des Innenkatheters beschränken kann, ohne daß ein neuer Außenschlauch eingeführt werden muß. Gleichzeitig gewährleistet der dickere und steifere Außenschlauch einen festen Halt, eine stabilere Positionierung und eine erhöhte Drehstabilität des Kathetersystems.

Diese Eigenschaft des Außenschlauches kann nun weiterhin genutzt werden, wie in Fig. 3 a/b ersichtlich, die ein weiteres Prinzip der Erfindung zeigen. Da der Außenschlauch sich aufgrund seines größeren Umfangs bzw. Durchmessers an der Eintrittsöffnung in ein weiteres Organ abgestützt und somit auch nicht weiter in das Hohlorgan 3 vordringen kann, führt er, wenn er dennoch weiter vorgeschoben wird, mit dem jenseits der Eintrittsöffnung eingeführten Kathetersegment eine Kippbewegung um die Eintrittsöffnung in das Organ aus. Diese Kippbewegung wirkt als Drehachse, um die der Außenschlauch - und damit auch der Innenkatheter - geschwenkt werden kann. Das führt zu einem weiteren Freiheitsgrad in der Bewegung mit einem festen Halt an der Drehachse, so daß stufenlos praktisch alle Punkte eines dreidimensionalen Raumes - z. B. eines Hohlorgans - erreicht werden können.

Die Verwendungsweise der Katheteranordnung sei nachfolgend kurz erläutert:
1. Nach Punktion eines Blutgefäßes, einer Vene oder Arterie, wird ein Führungsdraht gelegt (Seldinger Technik).
2. Zuerst wird der dünnere, durch einen in sein Lumen eingeführten Dilatator gestreckte, und weil längere, aus dem Außenschlauch überstehende Innenkatheter über den Führungsdraht in das Blutgefäß eingeführt.
3. Nach Einführung des überstehenden Segmentes des Innenkatheters wird duch Dehnung der elastischen Hautschichten und Gefäßwand auch der dickere, über den Innenkatheter reitende Außenschlauch in das Blutgefäß eingeführt und gleichzeitig mit dem Innenkatheter vorgeschoben, bis die Katheterspitze das angesteuerte Herz- oder Gefäßsegment erreicht hat.
4. Dann werden der Dilatator und der Führungsdraht entfernt und der Innenkatheter kann jetzt seine vorgegebenen Krümmungen annehmen.
5. Durch das Arbeitslumen des Innenkatheters können nun die gewünschten Werkzeuge wie Laserkatheter, Elektrodenkatheter, Biopsziezange oder dgl. vorgeschoben werden.
6. Durch das Zurückziehen des Innenkatheters in den Außenschlauch oder durch dessen Vorschieben über den Innenkatheter können die Krümmungen des Endabschnittes nach Bedarf variiert werden.
7. Zusätzlich kann der Innenschlauch mit oder ohne den Außenschlauch um seine eigene Achse gedreht werden.
8. Ist der Innenschlauch in ein Organ, z.B. eine andere Herzhöhle, eingeführt (z.B. transseptale Punktion), kann das distale Ende des Außenschlauches bis an das Organ geführt und dort wegen seines größeren Umfangs an der Eintrittsstelle abgestützt werden.
9. Jetzt können die oben beschriebenen Bewegungen mit dem Innenkatheter mit Kippbewegungen des Innenkatheters um die Achse an der Eintrittsöffnung des Organs kombiniert werden, wobei sich diese Kipp-/Schaukelbewegung aus dem Vorschub des Außenschlauchs ergibt.

Durch die erfindungsgemäße Katheteranordnung wird der Katheter nicht wie im Stand der Technik entlang des vorgegebenen Verlaufes eines Blutgefäßes über einen Führungsdraht manövriert, sondern durch das Vorschieben bzw. Zurückziehen des äußeren Katheters oder Außenschlauches über den vorgeformten inneren Katheter wird die Orientierung des distalen Segmentes oder Endabschnittes des Innenkatheters im Hohlraum (Herzen) verändert. Dadurch kann das im Innenlumen des Innenkatheters geführte Arbeitsinstrument (Lichtleiter, Biopsziezange usw.) kontinuierlich stufenlos und kontrolliert bewegt und eingesetzt werden, d. h. der vorgeformte aber flexible innere Plastikkatheter führt durch seine stufenlos verstellbaren Krümmungen das in seinem zentralen Lumen befindliche Instrument, das somit an beliebige Stellen des Hohlorgans (Herzen) geführt werden kann.

## Patentansprüche

1. Katheteranordnung aus Kunststoff, mit einem Außenkatheter (2) und einem darin angeordneten, längsverschieblichen Innenkatheter (1), der ein Arbeitslumen aufweist zur Aufnahme verschiedener Arbeitswerkzeuge, **dadurch gekennzeichnet,** daß zur verbesserten Steuerbarkeit des Innenkatheters der Außenkatheter eine größere Biegesteifigkeit aufweist als der Innenkatheter und der im normalen Gebrauchszustand über das distale Ende des Außenschlauches axial vorstehende distale Endabschnitt des Innenkatheters gekrümmt vorgeformt ist mit einem sich in Längsrichtung verändernden Krümmungsradius, wobei die vorgeformte Krümmung des distalen Endabschnittes in mindestens einer Krümmungsebene vorgesehen ist und sich beim Zurückziehen des Innenkatheters in den Außenkatheter an die jeweilige Krümmung des Außenkatheters anpaßt.

2. Katheteranordnung nach Anspruch 1, **dadurch gekennzeichnet,** daß die vorgeformte Krümmung des distalen Endabschnittes in mindestens zwei Krümmungsebenen vorgesehen ist, die sich unter einem Winkel größer als Null schneiden.

3. Katheteranordnung nach Anspruch 2, **dadurch gekennzeichnet,** daß zwei Krümmungsebenen vorgesehen sind, die zueinander senkrecht stehen.
